Europäisches Patentamt

European Patent Office

Office européen des brevets

(19)

(11) **EP 0 952 816 B1**

(12) **FASCICULE DE BREVET EUROPEEN**

(45) Date de publication et mention
de la délivrance du brevet:
**20.11.2002 Bulletin 2002/47**

(51) Int Cl.⁷: **A61K 7/48**, A61K 35/78,
A61K 7/06

(21) Numéro de dépôt: **97936755.4**

(22) Date de dépôt: **07.08.1997**

(86) Numéro de dépôt international:
**PCT/FR97/01468**

(87) Numéro de publication internationale:
**WO 98/010740 (19.03.1998 Gazette 1998/11)**

(54) **UTILISATION COSMETIQUE, DERMATOLOGIQUE ET PHARMACEUTIQUE D'UN EXTRAIT DE TERMINALIA CATAPPA**

KOSMETISCHE, DERMATOLOGISCHE UND PHARMAZEUTISCHE ANWENDUNG EINES
TERMINALIA CATAPPA PFLANZENEXTRAKTS

COSMETIC, DERMATOLOGICAL AND PHARMACEUTICAL USE OF A TERMINALIA CATAPPA
EXTRACT

(84) Etats contractants désignés:
**AT BE CH DE DK ES FI FR GB GR IE IT LI LU MC
NL PT SE**

(30) Priorité: **16.09.1996 FR 9611403**

(43) Date de publication de la demande:
**03.11.1999 Bulletin 1999/44**

(73) Titulaire: **Cognis France S.A.
31360 Saint Martory (FR)**

(72) Inventeur: **PAULY, Gilles
F-54000 Nancy (FR)**

(74) Mandataire: **Nuss, Pierre
Cabinet Nuss
10, rue Jacques Kablé
67080 Strasbourg Cédex (FR)**

(56) Documents cités:
- **STN, Serveur de Bases de Données,
XP002028786**
- **STN, Serveur de Bases de Données,
XP002028787**
- **STN, Serveur de Bases de Données,
XP002028788**

**Description**

La présente invention concerne les domaines de la cosmétologie et de la dermatologie, et a pour objet l'utilisation pour des applications cosmétiques, dermatologiques ou pharmaceutiques d'un extrait de la plante Terminalia catappa, ainsi qu'une composition cosmétique, dermatologique ou pharmaceutique correspondante.

La plante Terminalia catappa ou "badamier des Indes" est une espèce très commune, d'origine indienne et de la famille des Combrétacées.

Il s'agit d'un arbre de 5 à 25 m de haut, dont les branches sont horizontales et subverticillées, surtout au sommet des jeunes plants.

Les feuilles sont alternes, entières, membraneuses et distinctes en fonction de l'âge.

La défeuillaison s'effectue à la fin de la saison sèche, les feuilles virant au rouge avant leur chute.

La répartition géographique large, tropicale et subtropicale, de la plante Terminalia catappa explique partiellement son utilisation importante dans les médecines populaires africaines, asiatiques et américaines, ce depuis de nombreuses années.

En usage externe, la plante Terminalia catappa est surtout utilisée dans les soins des affections dermatologiques et rhumatismales.

En usage interne, cette plante intervient dans le traitement des affections gastro-intestinales, respiratoires et cardio-vasculaires.

Toutes les parties de la plante (feuilles, écorce, racines, fruit, bois) sont utilisées en médecine traditionnelle.

L'extrait d'écorce de tiges est utilisé par voie externe pour son action astringente (tannins) en cas de plaies et d'ulcérations.

Néanmoins, ce sont surtout les feuilles, seules ou en association avec d'autres plantes, qui sont utilisées dans le traitement des affections dermatologiques et rhumatismales.

Le jus obtenu après pressage des feuilles est utilisé dans le traitement de la gale, de la lèpre, du prurigo et des dartres (Sénégal) et dans le traitement des plaies (Madagascar, Nouvelle Guinée).

En Inde, la plante a été utilisée dans les systèmes de médecine Ayurvédique et Unani : le jus des feuilles jeunes est utilisé dans le traitement des gales et autres désordres cutanés. Un effet hémostatique est parfois mentionné.

Les feuilles peuvent être appliquées sur les articulations douloureuses engendrant un effet analgésique (Caraïbes, Sénégal, Indochine, Malaisie).

Sous formes de compresses locales, les feuilles vertes écrasées et enduites d'huile de ricin sont utilisées pour soulager le lumbago.

Associée à d'autres plantes et à un véhicule huileux, elle est utilisée pour les soins des entorses et/ou douleurs musculaires (Iles COOK).

En usage interne, les feuilles de Terminalia catappa sont utilisées pour le traitement des douleurs gastriques (Sénégal), des affections respiratoires (Caraïbes, Haïti, Mexique, Cuba, Nouvelle Guinée), des affections cardio-vasculaires (action anti-hypertensive) et hépatiques (médecine créole), de l'insomnie (médecine créole) et de la blennorragie (Madagascar), ou encore comme anti-diarrhéique (Indochine, Inde).

Il a d'autre part été montré, sur des cellules hépatiques isolées, que les extraits de feuilles sont dotés, in vitro, de propriétés anti-lipidoperoxydantes et anti-hépato-toxiques.

L'activité anti-radicalaire des extraits a également été démontrée in vitro par le test de décoloration du radical diphényl-picryl-hydrazyl (DPPH) (JOYEUX M. et al., PHYTOTHERAPY RESEARCH, 1995, 9,228-230).

Par ailleurs, on connaît également par différents documents l'utilisation en cosmétique d'extraits des espèces Terminalia chebula et Terminalia bellerica pour le traitement de désordres cutanés, en tant qu'actifs anti-hyaluronidase, en tant qu'actifs inhibiteurs de la 5α- réductase ou en tant qu'actifs anti-oxydants (voir notamment GB-2 274 058, JP-0 6009371, JP-0 5255102 et JP-0 4069343).

Enfin, il est également connu d'utiliser en tant qu'anti-inflammatoires, des complexes formés par des saponines végétales extraites de Terminalia sericea combinés avec des phospholipides (voir notamment US - 5 166 139).

Toutefois, une utilisation d'extraits de Terminalia catappa, pour une application topique externe, dans le domaine cosmétique et/ou pharmaceutique n'est pas encore connue.

Or, l'inventeur de la présente invention a constaté, de manière inattendue et surprenante, que les extraits de Terminalia catappa présentent des activités biologiques spécifiques, qui les rendent directement utilisables dans des préparations ou compositions cosmétiques, dermatologiques et pharmaceutiques à application topique externe.

Ainsi, le principal objet de la présente invention consiste en l'utilisation d'un extrait de feuilles de la plante Terminalia catappa, obtenu par extraction à chaud et à reflux avec de l'eau, un alcool et/ou une cétone ou avec un mélange de ces différents solvants, en tant qu'agent actif présentant simultanément des actions anti-inflammatoire, apaisante, astringente, raffermissante, protectrice et réparatrice anti-stress prononcées, pour la préparation d'une composition cosmétique à usage topique externe pour la peau et les phanères.

L'obtention et la préparation de l'extrait précité peuvent être réalisés par différentes méthodes connues au moins en partie de l'homme du métier.

A titre d'exemples non limitatifs, on décrira ci-après différents procédés d'obtention possibles d'un extrait de Terminalia catappa utilisé dans le cadre de la présente invention.

Exemple I :

On prend 100 grammes de feuilles de Terminalia catappa et on les introduit dans des récipients du type Erlenmeyer, après les avoir grossièrement contusées.

On verse ensuite un litre d'eau distillée, portée à ébullition sous reflux, sur lesdites feuilles et on laisse infuser / macérer ce mélange dans une étuve à 37° C pendant 24 heures.

Le macérat est ensuite successivement filtré sur papier du type Whatman, concentré sous vide au moyen d'un évaporateur rotatif et, enfin, concentré sous courant d'azote jusqu'à formation d'un extrait sec.

Le rendement de cette extraction est de 12,6 % en poids par rapport aux feuilles initiales.

Exemple II :

On prend 100 grammes de feuilles de Terminalia catappa et on les traite conformément à l'exemple I, le solvant d'extraction étant de l'alcool à 90°, et non pas de l'eau (comme dans l'exemple I).

Le rendement de cette extraction est de 6,4 % en poids par rapport aux feuilles initiales.

Exemple III :

On introduit deux litres d'eau distillée dans un réacteur et on ajoute 200 grammes de feuilles de Terminalia catappa broyées.

On procède ensuite à une extraction sous agitation pendant une heure à environ 80 à 90° C, le liquide résultant étant, après refroidissement à température ambiante, centrifugé ou filtré pour séparer l'extrait.

On obtient ainsi 1,57 litres d'un extrait brun foncé contenant 3,50 % en poids d'extrait sec.

Le culot humide est extrait dans les mêmes conditions au moyen de deux litres d'eau et on récupère ainsi 1,73 litres d'un extrait brun, contenant 0,80 % en poids d'extrait sec.

Les deux extraits précités sont atomisés et on récupère 33,46 grammes de poudre pour le premier extrait et 11,6 grammes de poudre pour le second extrait, ce qui correspond à un rendement total de l'extraction de 22,5 % en poids par rapport aux feuilles.

Exemple IV :

On introduit un litre d'éthanol absolu dans un réacteur et on ajoute 200 grammes de feuilles de Terminalia catappa broyées.

On procède ensuite à une extraction par reflux sous agitation pendant une heure, le liquide résultant étant, après refroidissement à température ambiante, filtré pour séparer l'extrait de couleur vert foncé.

On procède ensuite à une extraction dans les mêmes conditions du culot au moyen d'un litre d'éthanol, ce dernier étant ensuite évaporé sous vide à 35-40° C et le résidu obtenu étant séché en étuve à 40-50° C.

Ce procédé permet d'obtenir 36,84 grammes de pâte à partir du premier extrait et 15,2 grammes à partir du second extrait, ce qui correspond à un rendement total d'extraction de 26 % en poids par rapport aux feuilles.

Les extraits de Terminalia catappa obtenus par l'intermédiaire des procédés décrits ci-dessus contiennent notamment des flavonoïdes, des polyphénols, des saponosides, des triterpènes, des diterpènes, des tanins catéchiques et des dérivés tanoïdes.

En vue de mettre en évidence en particulier l'activité anti-inflammatoire des extraits de la plante Terminalia catappa, l'inventeur a procédé à différentes expériences dont les conditions de mise en oeuvre et les résultats obtenus sont décrits ci-après.

Pour la réalisation de ces expériences, on a utilisé des rats Sprague - Dawley (Iffa Credo) pesant environ de 150 à 250 g. Ces animaux étaient placés en stabulation pendant les 15 jours qui ont précédé l'expérimentation, dans des cages en macrolon, l'animalerie étant cyclée et thermostatée (12 h de jour/ 12 h de nuit ; 20 à 22°C).

Avant tout traitement, le volume de la patte postérieure droite de chaque rat a été mesuré (vo) à l'aide d'un pléthysmomètre (Ugo Basile 7150, Apelex).

Les produits à tester étaient administrés par voie intrapéritonéale à une dose correspondant à 400 mg de plante sèche/kg, sous un volume de 1 ml/kg et les lots témoins recevaient du sérum physiologique en intrapéritonéale.

Chaque rat recevait ensuite une injection, dans le coussinet de la patte postérieure droite, de 0,05 ml de carragénine diluée à 1 % dans de l'eau déionisée.

Puis, le volume de la patte était mesuré (vt) à l'acmée de l'inflammation, soit trois heures après l'injection de l'agent inflammatoire.

Un test de Randall-Selitto était pratiqué lorsque l'extrait s'avérait suffisamment actif. Ce test était opéré à l'aide d'un analgésimètre à pression, permettant de déterminer le seuil douloureux en excerçant une force à l'aide d'un cône en téflon au niveau de la patte postérieure droite.

Les extraits injectés en intrapéritonéale correspondaient aux extraits aqueux et éthanoliques préparés selon les exemples I et II précités et les doses testées équivalaient, en terme d'extrait brut, à 50,4 mg d'extrait brut/kg pour l'extrait aqueux et à 25,6 mg d'extrait brut/kg pour l'extrait éthanolique.

Les pourcentages moyens d'inhibition de l'oedème ont été calculés aux différents relevés à partir des différences obtenues entre les volumes moyens mesurés avant (vo) et après (vt) l'injection de la carragénine selon la formule suivante :

$$\frac{[(vt - vo)\ \text{témoin} - (vt - vo)\ \text{traité}\ ] \times 100}{(vt - vo)\ \text{témoin}}$$

Pour le test de Randall-Selitto, le seuil douloureux observé (retrait de la patte ou cri) a été exprimé en grammes.

Les comparaisons statistiques ont été réalisées à l'aide du test ANOVA suivi d'un test t non apparié pour le test de l'oedème à la carragénine, et par le test non paramétrique de Mann-Whithney suivi d'un test t non apparié pour le test de Randall-Selitto.

Les expériences décrites ci-dessus ont conduit aux résultats exposés dans les tableaux ci-après :

Test de l'oedème à la carragénine

| Rats témoins | | | |
|---|---|---|---|
| N° rats | Vo | V 3h | Δ V |
| 1 | 1,85 | 3,30 | 1,45 |
| 2 | 1,90 | 3,27 | 1,37 |
| 3 | 1,87 | 3,25 | 1,38 |
| 4 | 1,87 | 3,27 | 1,40 |
| Moyenne | 1,87 | 3,27 | 1,40 |
| SEM | 0,01 | 0,01 | 0,02 |

| Rats traités (extrait éthanolique) | | | | |
|---|---|---|---|---|
| N° rats | Vo | V 3h | ΔV | % inhibition |
| 5 | 1,84 | 2,50 | 0,66 | |
| 6 | 1,91 | 2,58 | 0,67 | |
| 7 | 1,85 | 2,53 | 0,68 | |
| 8 | 1,87 | 2,54 | 0,67 | |
| Moyenne | 1,87 | 2,54 | 0,67* | 52,11 |
| SEM | 0,02 | 0,02 | 0,00 | |

| Rats traités (extrait aqueux) | | | | |
|---|---|---|---|---|
| N° rats | Vo | V 3h | ΔV | % inhibition |
| 9 | 1,80 | 2,51 | 0,71 | |
| 10 | 1,90 | 2,55 | 0,65 | |
| 11 | 1,75 | 2,67 | 0,92 | |
| 12 | 1,82 | 2,58 | 0,76 | |

(suite)

| Rats traités (extrait aqueux) | | | | |
|---|---|---|---|---|
| N° rats | Vo | V 3h | $\Delta$V | % inhibition |
| Moyenne | 1,82 | 2,58 | 0,76** | 45,66 |
| SEM | 0,03 | 0,03 | 0,06 | |

*différence significative par rapport au témoin : p = 0,0001
** différence significative par rapport au témoin : p = 0,0001
Test ANOVA suivi de test t non apparié

Test de Randall-Selitto

### Rats témoins

| N° rat | Seuil douloureux (g) |
|---|---|
| 1 | 80 |
| 2 | 65 |
| 3 | 70 |
| 4 | 55 |
| Moyenne | 67,50 |
| SD | 10,41 |
| SEM | 3,29 |

### Rats traités par un extrait de Terminalia catappa

| N° rat | Seuil douloureux (g) |
|---|---|
| 5 | 140 |
| 6 | 120 |
| 7 | 110 |
| 8 | 130 |
| Moyenne | 125,00*** |
| SD | 12,91 |
| SEM | 4,27 |

*** différence significative par rapport au témoin : p = 0,0004 (test de Mann-Whithney suivi d'un test t non apparié).

Il ressort clairement des résultats exposés dans les tableaux ci-dessus que les extraits de Terminalia catappa présentent notamment une activité anti-inflammatoire prononcée.

La présente invention a également pour objet une composition cosmétique à usage topique pour la peau et/ou les phanères, caractérisée en ce qu'elle contient notamment, à titre d'agent actif à actions anti-inflammatoire, apaisante, astringente, raffermissante, protectrice et réparatrice anti-stress, une quantité cosmétiquement efficace d'extrait de feuilles de la plante Terminalia catappa, obtenu par extraction à chaud et à reflux avec de l'eau, un alcool et/ou une cétone ou avec un mélange de ces différents solvants.

Les activités cosméto-dynamiques ou, le cas échéant, thérapeutiques exercées par ces compositions ou préparations comprenant ces extraits consistent donc en des actions prononcées anti-inflammatoires locales, apaisantes, raffermissantes et astringentes au niveau de la peau, anti-vieillissement de la peau et anti-irritantes au niveau de la peau et du cuir chevelu.

Conformément à un mode de réalisation préférentiel de l'invention, la composition cosmétique, dermatologique ou pharmaceutique comporte entre 0,10 % et 3 % en poids, d'extrait de la plante Terminalia catappa, obtenu par exemple au moyen de l'un des quatre procédés d'extraction décrits précédemment.

Lesdits extraits pourront être utilisés indifféremment soit tels quels (liquides, pâteux, secs), soit après préparation sous forme de microsphéres, microcapsules, nanocapsules, liposomes ou d'autres formes analogues connues.

Les compositions ou préparations cosmétiques, dermatologiques ou pharmaceutiques précitées pourront prendre l'une quelconque des formes galéniques utilisées habituellement pour ce type de produits, à savoir notamment des gels, des hydrogels, des crèmes, des pommades, des laits, des shampooings, des lotions capillaires, des savons, des émulsions H/E ou E/H, des émulsions multiples, des maquillages ou autres.

A titre d'exemples non limitatifs de réalisations pratiques de l'invention, on décrira ci-après différent(e)s produits ou préparations cosmétiques ou dermatologiques, comprenant un extrait de la plante Terminalia catappa.

Exemple 1:

Un produit cosmétique sous forme d'émulsion raffermissante et apaisante cutanée conforme à l'invention peut, par exemple, présenter une composition pondérale telle qu'indiquée ci-après (association des fractions A et B):
FRACTION A:

- Alcool cétylique        5,000
- Triglycéride caprylique / caprique        9,000
- Huile de paraffine        3,750
- Lanoline        1,000
- Stéarate de glycérol        2,000
- Diméthicone        0,250
- Octyldodécanol        4.500
- Stéarate d'octyle        12,000
- Elestab ® 4121 (Laboratoires Sérobiologiques)        0,300

FRACTION B:

- Eau        55,800
- Elestab ® 4112 (Laboratoires Sérobiologiques)        0,400
- Extrait de Terminalia Catappa (selon le procédé d'obtention de l'exemple I)        3,000
- Cétylphosphate de potassium        3,000

Le procédé de préparation de l'émulsion précitée consiste à chauffer séparément les fractions A et B à 80° C, puis à introduire la fraction A dans la fraction B à 80° C et sous agitation turbine, à refroidir ensuite le mélange jusqu'à 50° C en maintenant l'agitation turbine, et enfin à mettre le mélange sous agitation planétaire à 50° C et à le refroidir jusqu'à température ambiante.

Exemple 2 :

Un produit cosmétique sous forme d'émulsion capillaire conforme à l'invention peut, par exemple, présenter une composition pondérale telle qu'indiquée ci-après (association des fractions A, B et C) :
FRACTION A :

- Stéarate de glycérol (et) stéarate de PEG 100        2,000
- Alcool cétostéarylique (et) méthosulfate de dipalmitoyléthylhydroxyéthylammonium        2,500
- Alcool cétylique        3,000
- Diméthicone        2,000

FRACTION B:

- Eau        87,300
- Elestab ® 4112 (Laboratoires Sérobiologiques)        0,400
- Chlorure d'hydroxypropyltrimonium / hydroxypropyle guar gome        0,500
- Extrait de Terminalia Catappa (selon le procédé d'obtention de l'exemple II)        2,000

FRACTION C:

- Parfum        0,300

Le procédé de préparation de l'émulsion capillaire précitée consiste à chauffer séparément les fractions A et B à 75° C, puis à introduire la fraction A dans la fraction B à 75° C et sous agitation turbine, à refroidir ensuite le mélange jusqu'à 60° C en maintenant l'agitation turbine, à mettre le mélange sous agitation planétaire à 60° C et à poursuivre le refroidissement, à ajouter la fraction C à 40° C et, enfin, à continuer le refroidissement jusqu'à températue ambiante.

Exemple 3 :

Un produit cosmétique sous forme d'émulsion apaisante et réparatrice, pour application après exposition au soleil, conforme à l'invention, peut, par exemple, présenter une composition pondérale telle qu'indiquée ci-après (association des fractions A, B et C) :

FRACTION A :

- Stéarate de glycérol (et) stéarate de PEG 100     1,500
- Alcool cétostéarylique (et) Ceteh-20     1,500
- Alcool cétylique     1,000
- Triglycéride caprylique / caprique     8,000
- Isononanoate cétostéarylique     4,000
- Octyldodécanol     3,000
- Diméthicone     0,500
- Elestab ® 4121 (Laboratoires Sérobiologiques)     0,300

FRACTION B :

- Eau     73,550
- Elestab ® 4112 (Laboratoires Sérobiologiques)     0,400
- Extrait de Terminalia Catappa (selon le procédé d'obtention de l'invention III, dilué à 10 % dans du butylène glycol)     5,000
- Gomme xanthane     0,500

FRACTION C :

- Polyacrylamide (et) isoparaffine (et) Laureth-7     0,750

Le procédé de préparation de l'émulsion précitée consiste à turbiner tout d'abord la fraction B avant mise en émulsion, puis à chauffer séparément les fractions A et B à 80° C, à introduire la fraction A dans la fraction B à 80° C et sous agitation turbine, à refroidir le mélange obtenu à 60° C en maintenant l'agitation turbine, à ajouter ensuite la fraction C à 60° C sous agitation turbine et à continuer le refroidissement et, enfin, à refroidir jusqu'à température ambiante en réalisant une agitation au planétaire à partir de 50° C.

Exemple 4 :

Un produit cosmétique sous forme d'émulsion apaisante et astringente pour le visage (peaux sensibles) conforme à l'invention peut, par exemple, présenter une composition pondérale telle qu'indiquée ci-après (association des fractions A et B) :

FRACTION A :

- Ester palmitique de sorbitanne     3,500
- Stéarate de glycérol     1,500
- Alcool cétylique     2,500
- Isononanoate cétostéarylique     7,000
- Octyldodécanol     5,500
- Huile de parrafine     3,000
- Diméthicone     2,000
- Elestab ® 4121 (Laboratoires Sérobiologiques)     0,300

FRACTION B :

- Eau    69,100
- Elestab ® 4112 (Laboratoires Sérobiologiques)    0,400
- Extrait deTerminalia Catappa (selon le procédé d'obtention de l'exemple I, dilué à 20 % dans du propylène glycol)    4,000
- Sulfate cétostéarylique de sodium    1,200

Le procédé de préparation de l'émulsion précitée consiste à préparer séparément les fractions A et B à 80° C, puis à introduire la fraction A dans la fraction B à 80° C et sous agitation turbine, à refroidir ensuite le mélange obtenu à 50° C en maintenant l'agitation turbine, et, enfin, à refroidir jusqu'à température ambiante en réalisant une agitation au planétaire à partir de 50° C.

Exemple 5 :

Un produit cosmétique sous forme de gel protecteur astringent contour de l'oeil, conforme à l'invention, peut, par exemple, présenter une composition pondérale telle qu'indiquée ci-après (association des fractions A, B et C):
FRACTION A :

- Eau    70,925
- Stabilisateur LS 48 (Laboratoires Sérobiologiques)    0,225
- Extrait de Terminalia Catappa (selon le procédé d'obtention de l'exemple I)    0,100

FRACTION B :

- Eau    24,175
- Stabilisateur LS 48 (Laboratoires Sérobiologiques)    0,075
- Carbomer    0,750

FRACTION C :

- Hydroxyde de sodium (N)    3,750

Le procédé de préparation du gel précité consiste à préparer, dans un premier temps, séparément les fractions A et B, puis à réaliser, dans un deuxième temps, la mise en gel.
La préparation de la fraction A consiste à dissoudre le stabilisateur LS 48 dans de l'eau distillée à 50° C, à y disperser ensuite l'extrait de Terminalia catappa sous agitation turbine et à laisser refroidir le mélange résultant sous agitation planétaire jusqu'à température ambiante.
La préparation de la fraction B consiste à dissoudre le stabilisateur LS 48 dans de l'eau distillée à 50° C, à y disperser ensuite le Carbomer sous agitation turbine et à laisser refroidir le mélange résultant sous agitation planétaire jusqu'à température ambiante.
Pour obtenir le gel final, on introduit, à température ambiante et sous agitation turbine, les phases B et C dans la phase A et ensuite on homogénéise le mélange par agitation planétaire.

Exemple 6 :

Un produit cosmétique sous forme de lotion apaisante après soleil, conforme à l'invention, peut, par exemple, présenter une composition pondérale telle qu'indiquée ci-après (association des fractions A, B et C) :
FRACTION A :

- Sulfate d'alcool dodécylique 39 C (alcool phtalate)    3,000
- Nonoxynol-14    0,600
- Parfum    0,100

FRACTION B :

- Propylène glycol    2,000
- Stabilisateur LS 48 (Laboratoires Sérobiologiques)    0,300
- Eau    84,260
- Triéthanolamine 20 % aq. Sol.    0,240

FRACTION C :

- Extrait de Terminalia Catappa (selon le procédé d'obtention de l'exemple IV, dilué à 10 % dans du propylène glycol) 9,500

Le procédé de préparation de la lotion précitée consiste à préparer séparément les fractions A et C à température ambiante et sous agitation, à préparer la fraction B à 50° C sous agitation et à la refroidir à température ambiante, à introduire la fraction A, puis la fraction C, dans la fraction B, à laisser reposer le mélange obtenu pendant 72 heures et, enfin, à filter ce dernier.

Bien entendu, l'invention n'est pas limitée aux modes de réalisation décrits. Des modifications restent possibles, notamment du point de vue de la constitution des divers éléments ou par substitution d'équivalents techniques, sans sortir pour autant du domaine de protection de l'invention.

**Revendications**

1. Utilisation d'un extrait de feuilles de la plante Terminalia catappa, obtenu par extraction à chaud et à reflux avec de l'eau, un alcool et/ou une cétone ou avec un mélange de ces différents solvants, en tant qu'agent actif présentant simultanément des actions anti-inflammatoire, apaisante, astringente, raffermissante, protectrice et réparatrice anti-stress prononcées, pour la préparation d'une composition cosmétique à usage topique externe pour la peau et les phanères.

2. Composition cosmétique à usage topique pour la peau et/ou les phanères, **caractérisée en ce qu'**elle contient notamment, à titre d'agent actif à actions anti-inflammatoire, apaisante, astringente, raffermissante, protectrice et réparatrice anti-stress, une quantité cosmétiquement efficace d'extrait de feuilles de la plante Terminalia catappa, obtenu par extraction à chaud et à reflux avec de l'eau, un alcool et/ou une cétone ou avec un mélange de ces différents solvants.

3. Composition selon la revendication 2, **caractérisée en ce qu'**elle comporte entre 0,10 % et 3 % en poids d'extrait de feuilles de la plante Terminalia catappa.

**Patentansprüche**

1. Verwendung eines Extraktes aus Blättern der Terminalia catappa-Pflanze, das durch Warm- und Rückflussextraktion mit Wasser, einem Alkohol und/oder einem Keton oder mit einer Mischung aus diesen unterschiedlichen Lösungsmitteln gewonnen wird, als Wirkstoff, der gleichzeitig ausgeprägte entzündungsstillende, beruhigende, adstringierende, kräftigende, schützende und stressmindernde Wirkungen aufweist, für die Herstellung einer kosmetischen Zusammensetzung zur äußerlichen örtlichen Anwendung für die Haut und die Hautanhangsgebilde.

2. Kosmetische Zusammensetzung zur örtlichen Anwendung für die Haut und/oder die Hautanhangsgebilde, **dadurch gekennzeichnet, dass** sie als Wirkstoff mit entzündungsstillenden, beruhigenden, adstringierenden, kräftigenden, schützenden und stressmindernden Wirkungen insbesondere eine kosmetisch wirksame Menge an Extrakt aus Blättern der Terminalia catappa-Pflanze enthält, das durch Warm- und Rückflussextraktion mit Wasser, einem Alkohol und/oder einem Keton oder mit einer Mischung aus diesen unterschiedlichen Lösungsmitteln gewonnen wird.

3. Zusammensetzung nach Anspruch 2, **dadurch gekennzeichnet, dass** sie zwischen 0,10 und 3 Gewichtsprozent eines Extraktes aus Blättern der Terminalia catappa-Pflanze enthält.

**Claims**

1. Use of a leaf extract from the *Terminalia catappa* plant obtained by heat extraction and reflux with water, an alcohol and/or a ketone or with a mixture of these various solvents, as an active agent simultaneously having pronounced anti-inflammatory, soothing, astringent, firming, protective and anti-stress repairing actions, for the preparation of a cosmetic composition for external topical use for the skin and superficial body growths.

2. Cosmetic composition for topical use for the skin and/or superficial body growths, **characterised in that** as an active agent with anti-inflammatory, soothing, astringent, firming, protective and anti-stress repairing actions, it contains a cosmetically effective quantity of leaf extract from the *Terminalia catappa* plant, obtained by heat extraction and reflux with water, an alcohol and/or a ketone or with a mixture of these various solvents.

3. Composition according to claim 2, **characterised in that** it comprises between 0.10 and 3 % by weight of leaf extract from the *Terminalia catappa* plant.